# EUROPEAN PATENT SPECIFICATION

(11) **EP 2 220 053 B1**
(45) Date of publication and mention of the grant of the patent: **11.05.2011**
(21) Application number: 08841729.0
(22) Date of filing: 23.10.2008
(51) Int. Cl.: C07D 239/70, C07D 403/06, C07D 401/06, A61K 31/517, A61P 25/16

(54) **ARYLINDENOPYRIMIDINES AND THEIR USE AS ADENOSINE A2A RECEPTOR ANTAGONISTS**
ARYLINDENOPYRIMIDINE UND IHRE VERWENDUNG ALS ADENOSIN-A2A-REZEPTORANTAGONISTEN
ARYLINDÉNOPYRIMIDINES ET LEUR UTILISATION EN TANT QU'ANTAGONISTES DE RÉCEPTEUR A2A D'ADÉNOSINE

(30) Priority: 25.10.2007 US 982456 P
(43) Date of publication of application: 25.08.2010
(73) Proprietor: Janssen Pharmaceutica, N.V., 2340 Beerse (BE)
(72) Inventor: SHOOK, Brian C., Gilbertsville Pennsylvania 19525 (US); JACKSON, Paul F., New Hope, Pennsylvania 18938 (US)
(74) Representative: Warner, James Alexander
(86) International application number: PCT/US2008/080923
(87) International publication number: WO 2009/055548

(56) References cited:
- WO-A-2005/042500
- JULIUS J. MATASI ET AL: "The discovery and synthesis of novel adenosine receptor (A2A) antagonists" BIOORGANIC & MEDICINAL CHEMISTRY LETTERS, vol. 15, no. 5, 1 March 2005 (2005-03-01), pages 1333-1336, XP004750663 ISSN: 0960-894X

## Description

### FIELD OF THE INVENTION

This invention relates to novel arylindenopyrimidines and their therapeutic and prophylactic uses. Disorders treated and/or prevented using these compounds include neurodegenerative and movement disorders ameliorated by antagonizing Adenosine A2a receptors. The present application is directed to a subset of a pending genus of compounds, disclosed in applications US 20040127510(A1), and WO 2005/042500 A1.

### BACKGROUND OF THE INVENTION

Adenosine is a purine nucleotide produced by all metabolically active cells within the body. Adenosine exerts its effects via four subtypes of cell surface receptors (A1, A2a, A2b and A3), which belong to the G protein coupled receptor superfamily (Stiles, G.L. Journal of Biological Chemistry, 1992, 267, 6451). A1 and AS couple to inhibitory G protein, while A2a and A2b couple to stimulatory G protein. A2a receptors are mainly found in the brain, both in neurons and glial cells (highest level in the striatum and nucleus accumbens, moderate to high level in olfactory tubercle, hypothalamus, and hippocampus etc. regions) (Rosin, D. L.; Robeva, A.; Woodard, R. L.; Guyenet, P. G.; Linden, J. Journal of Comparative Neurology,1998, 401, 163).

In peripheral tissues, A2a receptors are found in platelets, neutrophils, vascular smooth muscle and endothelium (Gessi, S.; Varani, K. ; Merighi, S. ; Ongini, E.; Bores, P. A. British Journal of Pharmacology, 2000, 129, 2). The striatum is the main brain region for the regulation of motor activity, particularly through its innervation from dopaminergic neurons originating in the substantial nigra. The striatum is the major target of the dopaminergic neuron degeneration in patients with Parkinson's Disease (PD). Within the striatum, A2a receptors are co-localized with dopamine D2 receptors, suggesting an important site for the integration of adenosine and dopamine signaling in the brain (Fink, J. S.; Weaver, D. Ri; Rivkees, S. A.; Peterfreund, R. A.; Pollack, A. E.; Adler, E. M.; Reppert, S. M. Brain Research Molecular Brain Research, 1992,14,186).

Neurochemicat studies have shown that activation of A2a receptors reduces the binding affinity of D2 agonist to their receptors. This D2R and A2aR receptor-receptorinteraction has been demonstrated instriatal membrane preparations of rats (Ferre, S.; con Euler, G.; Johansson, B.; Fredholm, B. B.; Fuxe, K. Proceedings of the National Academy of Sciences I of the United States of America, 1991, 88, 7238) as well as in fibroblast cell lines after transfected with A2aR and D2R cDNAs (Salim, H. ; Ferre, S.; Dalal, A.; Peterfreund, R. A.; Fuxe, K.; Vincent, J. D.; Lledo, P. M. Journal of Neurochemistry, 2000, 74, 432). In viva, pharmacological blockade of A2a receptors using A2a antagonist leads to beneficial effects in dopaminergic neurotoxin MPTP(1-methyl-4-pheny-1,2,3,6-tetrahydropyridine)-induced PC) in various species, including mice, rats, and monkeys (Ikeda, K.; Kurokawa, M.; Aoyana, S.; Kuwana, Y. Journal of Neurochemistry, 2002, 80, 262).

Furthermore, A2a knockout mice with genetic blockade of A2a function have been found to be less sensitive to motor impairment and neurochemical changes when they were exposed to neurotoxir MPTP (Chen, J. F.; Xu, K.; I Petzer, J. P.; Steal, R.; Xu, Y. H.; Beilstein, M.; Sonsalla, P. K.; Castagnoli, K.; Castagnoli, N., Jr.; Schwarsschild, M. A. Journal of Neuroscience, 2001, 1 21, RC1 43).

In humans, the adenosine receptor antagonist theophylline has been found to produce beneficial effects in PD patients (Mally, J.; Stone, T. W. Journal of the Neurological Sciences, 1995, 132, 129). Consistently, recent epidemiological study has shown that high caffeine consumption makes people less likely to develop PD (Ascherio, A.; Zhang, S. M.; Hernan, M. A.; Kawachi, I.; Colditz, G. A.; Speizer, F. E.; Willett, W. C. Annals of Neurology, 2001, 50, 56). In summary, adenosine A2a receptor blockers may provide a new class of antiparkinsonian agents (Impagnatiello, F.; Bastia, E.; Ongini, E.; Monopoli, A. Emerging Therapeutic Targets, 2000, 4, 635).

Antagonists of the A_{2A} receptor are potentially useful therapies for the treatment of addiction. Major drugs of abuse (opiates, cocaine, ethanol, and the like) either directly or indirectly modulate dopamine signaling in neurons particularly those found in the nucleus accumbens, which contain high levels of A_{2A} adenosine receptors. Dependence has been shown to be augmented by the adenosine signaling pathway, and it has been shown that administration of an A_{2A} receptor antagonist redues the craving for addictive substances ("The Critical Role of Adenosine A2A Receptors and Gi βγ Subunits in Alcoholism and Addiction: From Cell Biology to Behavior", by Ivan Diamond and Lina Yao, (The Cell Biology of Addiction, 2006, pp 291-316) and "Adaptations in Adenosine Signaling in Drug Dependence: Therapeutic Implications", by Stephen P. Hack and Macdonald J. Christie, Critical Review in Neurobiology, Vol. 15, 235-274 (2003)). See also Alcoholism: Clinical and Experimental Research (2007), 31(8), 1302-1307.

An A_{2A} receptor antagonist could be used to treat attention deficit hyperactivity disorder (ADHD) since caffeine (a non selective adenosine antagonist) can be useful for treating ADHD, and there are many interactions between dopamine and adenosine neurons. Clinical Genetics (2000), 58(1), 31-40 and references therein.

Antagonists of the A_{2A} receptor are potentially useful therapies for the treatment of depression. A_{2A} antagonists are known to induce activity in various models of depression including the forced swim and tail suspension tests. The positive response is mediated by dopaminergic transmission and is caused by a prolongation of escape-directed behavior rather than by a motor stimulant effect. Neurology (2003), 61(suppl 6) S82-S87. Antagonists of the A_{2A} receptor are potentially useful therapies for the treatment of anxiety. A_{2A} antagonist have been shown to prevent emotional/anxious responses in vivo. Neurobiology of Disease (2007), 28(2) 197-205.

### SUMMARY OF THE INVENTION

Compounds **A, B**, and **C** are potent small molecule antagonists of the Adenosine A2a receptor.

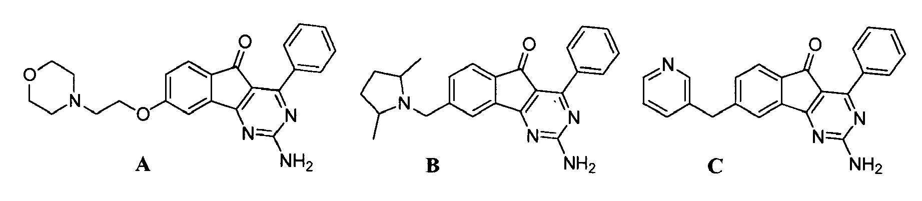

### DETAILED DESCRIPTION OF THE INVENTION

This invention provides compounds **A**, **B,** and **C**. 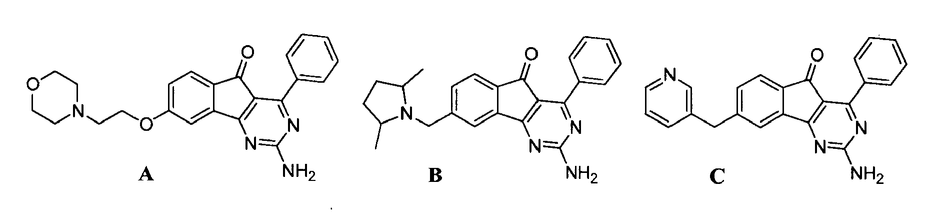 This invention further provides a method of treating a subject having a; condition ameliorated by antagonizing Adenosine A2a receptors, which comprises administering to the subject a therapeutically effective dose of the instant pharmaceutical composition.

This invention further provides a method of preventing a disorder ameliorated by antagonizing Adenosine A2a receptors in a subject, comprising of administering to the subject a prophylactically effective dose of the compound of claim 1 either preceding or subsequent to an event anticipated to cause a disorder ameliorated by antagonizing Adenosine A2a receptors in the subject.

The instant compounds can be isolated and used as free bases. They can also be isolated and used as pharmaceutically acceptable salts.

Examples of such salts include hydrobromic, hydroiodic, hydrochloric, perchloric, sulfuric, maleic, fumaric, malic, tartaric, citric, adipic, benzoic, mandelic, methanesulfonic; hydroethanesulfonic, benzenesulfonic, oxalic, palmoic, 2 naphthalenesulfonic, p-toluenesulfonic, cyclohexanesulfamic and saccharic.

This invention also provides a pharmaceutical composition comprising the instant compound and a pharmaceutically acceptable carrier.

Pharmaceutically acceptable carriers are well known to those skilled in the art and include, but are not limited to, from about 0.01 to about 0.1 M and preferably 0.05 M phosphate buyer or 0.8% saline. Such pharmaceutically acceptable carriers can be aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, ethanol, alcoholic/aqueous solutions, glycerol, emulsions or suspensions, including saline and buffered media. Oral carriers can be elixirs, syrups, capsules, tablets and the like. The typical solid carrier is an inert substance such as lactose, starch, glucose, methylcellulose, magnesium stearate, dicalcium phosphate, mannitol and the like. Parenteral carriers include sodium chloride solution, Ringer's dextrose, dextrose and sodium chloride, lactated Ringer's and fixed oils. Intravenous carriers include fluid and nutrient replenishers, electrolyte replenishers such as those based on Ringer's dextrose and the like.

Preservatives and other additives can also be present, such as, for example, antimicrobials, antioxidants, chelating agents, inert gases and the like. All carriers can be mixed as needed with disintegrants, diluents, granulating agents, lubricants, binders and the like using conventional techniques known in the art.

This invention further provides a method of treating a subject having a condition ameliorated by antagonizing Adenosine A2a receptors, which comprises administering to the subject a therapeutically effective dose of the instant pharmaceutical composition.

In one embodiment, the disorder is a neurodegenerative or movement disorder. Examples of disorders treatable by the instant pharmaceutical composition include, without limitation, Parkinson's Disease, Huntington's Disease, Multiple System Atrophy, Corticobasal Degeneration, Alzheimer's Disease, and Senile Dementia.

In one preferred embodiment, the disorder is Parkinson's disease.

As used herein, the term "subject" includes, without limitation, any animal or artificially modified animal having a disorder ameliorated by antagonizing adenosine A2a receptors. In a preferred embodiment, the subject is a human.

Administering the instant pharmaceutical composition can be effected or performed using any of the various methods known to those skilled in the art. The instant compounds can be administered, for example, intravenously, intramuscularly, orally and subcutaneously. In the preferred embodiment, the instant pharmaceutical composition is administered orally. Additionally, administration can comprise giving the subject a plurality of dosages over a suitable period of time. Such administration regimens can be determined according to routine methods.

As used herein, a "therapeutically effective dose" of a pharmaceutical composition is an amount sufficient to stop, reverse or reduce the progression of a disorder. A "prophylactically effective dose" of a pharmaceutical composition is an amount sufficient to prevent a disorder, i.e., eliminate, ameliorate and/or delay the disorder's onset. Methods are known in the art for determining therapeutically and prophylactically effective doses for the instant pharmaceutical composition. The effective dose for administering the pharmaceutical-composition to a human, for example, can be determined mathematically from the results of animal studies.

In one embodiment, the therapeutically and/or prophylactically effective dose is a dose sufficient to deliver from about 0.001 mg/kg of body weight to about 200 mg/kg of body weight of the instant pharmaceutical composition. In another embodiment, the therapeutically and/or prophylactically effective dose is a dose sufficient to deliver from about 0.05 mg/kg of body weight to about 50 mg/kg of body weight. More specifically, in one embodiment, oral doses range from about 0.05 mg/kg to about 100 mg/kg daily. In another embodiment, oral doses range from about 0.05 mg/kg to about 50 mg/kg daily, and in a further embodiment, from about 0.05 mg/kg to about 20 mg/kg daily. In yet another embodiment, infusion doses range from about 1.0,ug/kg/min to about 10 mg/kg/min of inhibitor, admixed with a pharmaceutical carrier over a period ranging from about several minutes to about several days. In a further embodiment, for topical administration, the instant compound can be combined with a pharmaceutical carrier at a drug/carrier ratio of from about 0.001 to about 0.1.

The invention also provides a method of treating addiction in a mammal, comprising administering a therapeutically effective dose of a compound of Formula **A,** Formula **B,** or Formula **C.**

The invention also provides a method of treating ADHD in a mammal, comprising administering a therapeutically effective dose of a compound of Formula **A,** Formula **B,** or Formula **C.**

The invention also provides a method of treating depression in a mammal, comprising administering a therapeutically effective dose of a compound of Formula **A,** Formula **B,** or Formula **C.**

The invention also provides a method of treating anxiety in a mammal, comprising administering a therapeutically effective dose of a compound of Formula **A,** Formula **B,** or Formula **C.**

### EXAMPLES:

Compounds **A, B and C** can be prepared by methods known to those who are skilled in the art. The following reaction schemes are only meant to represent examples of the invention and are in no way meant to limit the invention.

### Procedure

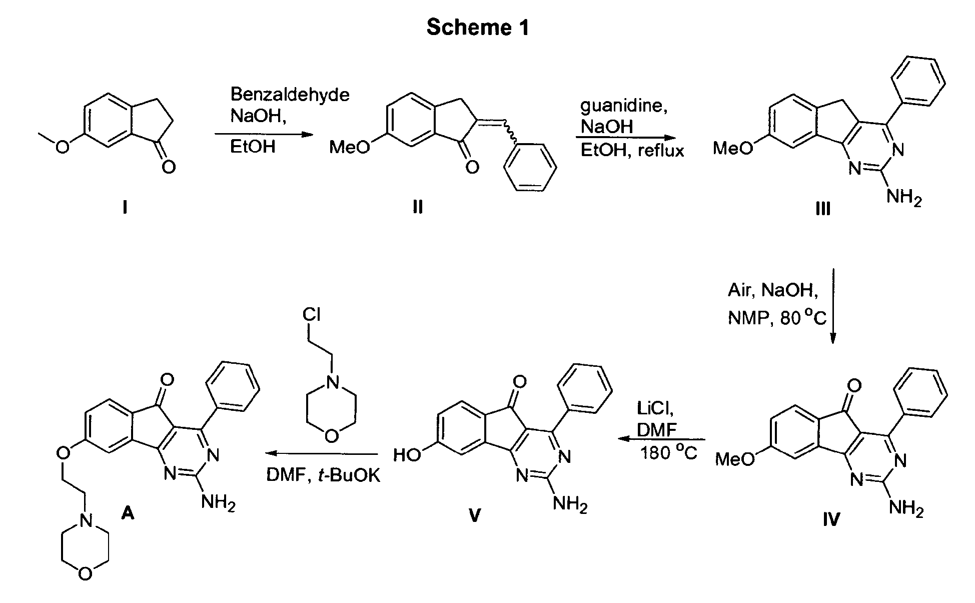

Scheme 1 illustrates the synthetic route leading to compound **A.** Starting with 6-methoxy indanone **I** and following the path indicated by the arrows, condensation under basic conditions with arylaldehydes affords the benzylidene **II.** The benzylidene **II** is then reacted with guanidine (free base) that gives the intermediate amino pyrimidine **III** and is directly oxidized to the corresponding ketone **IV** by bubbling air through the basic N-methyl pyrrolidinone (NMP) solution. Demethylation can be accomplished by heating **IV** in NMP in the presence of LiCl to give the corresponding phenol **V.** The phenol **V** can be directly converted to corresponding ether **A** by treatment with 4-(2-chloroethyl)morpholine hydrochloride under basic conditions in dimethylformamide (DMF).

### Example A: step a

**(II)** An aqueous solution (5 mL) of NaOH (1.5 g, 38.5 mmol) was added dropwise to an ethanol (EtOH) solution (30 mL) of **I** (5.0 g, 30.8 mmol) and benzaldehyde (3.4 g, 32.4 mmol). A precipitate formed immediately. The resulting slurry was stirred vigorously for 0.5 h. The slurry was cooled in an ice bath, filtered, and washed with cold EtOH. The collected solid was dried *in vacuo* to give 7.3 g of **II.**

### Example A: steps b and c

**(III** and **IV):** Powdered NaOH (5.8 g, 146.0 mmol) was added to an EtOH solution (130 mL) of guanidine acetate (17.4 g, 146.0 mmol). After 30 min the sodium acetate (NaOAc) was filtered off and the filtrate was added to an EtOH suspension (80 mL) of **II** (7.3 g, 29.2 mmol). The resulting mixture was heated to reflux overnight. The homogeneous solution was cooled in ice for 30 minutes and filtered to give 6.0 g of **III,** which was used without further purification. Powdered NaOH (1.2 g, 31.1 mmol) was added to a NMP solution (80 mL) of **III** (6.0 g, 20.8 mmol). The resulting mixture was heated to 80 °C and air was bubbled through the solution. After 16 h the mixture was cooled to rt, water was added and the resulting precipitate was filtered and washed with water and cold EtOH. The red solid was dried *in vacuo* to give 5.0 g of **IV.**

### Example A: step d

**(V):** A DMF solution (5 mL) of **IV** (1.0 g, 3.3 mmol), LiCl (840 mg, 19.8 mmol) and water (0.1 mL) were heated to 180 °C in the microwave. After 1 h the mixture was diluted with THF, filtered and dry packed onto silica gel. Chromatography gave 530 mg of **V.**

### Example A: step e

### 2-Amino-8-(2-morpholin-4-yl-ethoxy)-4-phenyl-indeno[1,2-d]pyrimidin-5-one

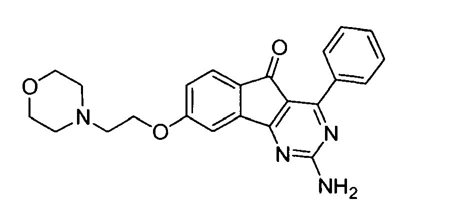

Solid potassium *tert*-butoxide (*t*-BuOK) (435 mg, 3.9 mmol) was added to a DMF solution (7.5 mL) of **V** (448 mg, 1.6 mmol) and 4-(2-chloroethyl)morpholine hydrochloride (318 mg, 1.7 mmol) and the resulting solution was heated to 75 °C. After 4 h at 75 °C the mixture was cooled to rt diluted with tetrahydrofuran (THF) and EtOAc and washed with brine, water, and brine. The organics were dried over Na₂SO₄ and dry packed onto silica gel. Column chromatography gave 404 mg of the desired ether as the free base, which was dissolved in THF and added to 5 mL of 1 N HCl in ether, concentrated, and dried *in vacuo* to give the title compound **(A)** as the di-HCl salt. ¹H NMR (300 MHz, CHLOROFORM-*d*) δ ppm 2.53 - 2.67 (m, 4 H), 2.86 (t, *J*=5.65 Hz, 2 H), 3.66 - 3.83 (m, 4 H), 4.25 (t, *J*=5.65 Hz, 2 H), 5.76 (br. s., 2 H), 7.01 (dd, *J*=8.10, 2.45 Hz, 1 H), 7.35 (d, *J*=2.26 Hz, 1 H), 7.43 - 7.57 (m, 3 H), 7.66 (d, *J*=8.29 Hz, 1 H), 7.98 - 8.10 (m, 2 H); MS m/e 403 (M+H). 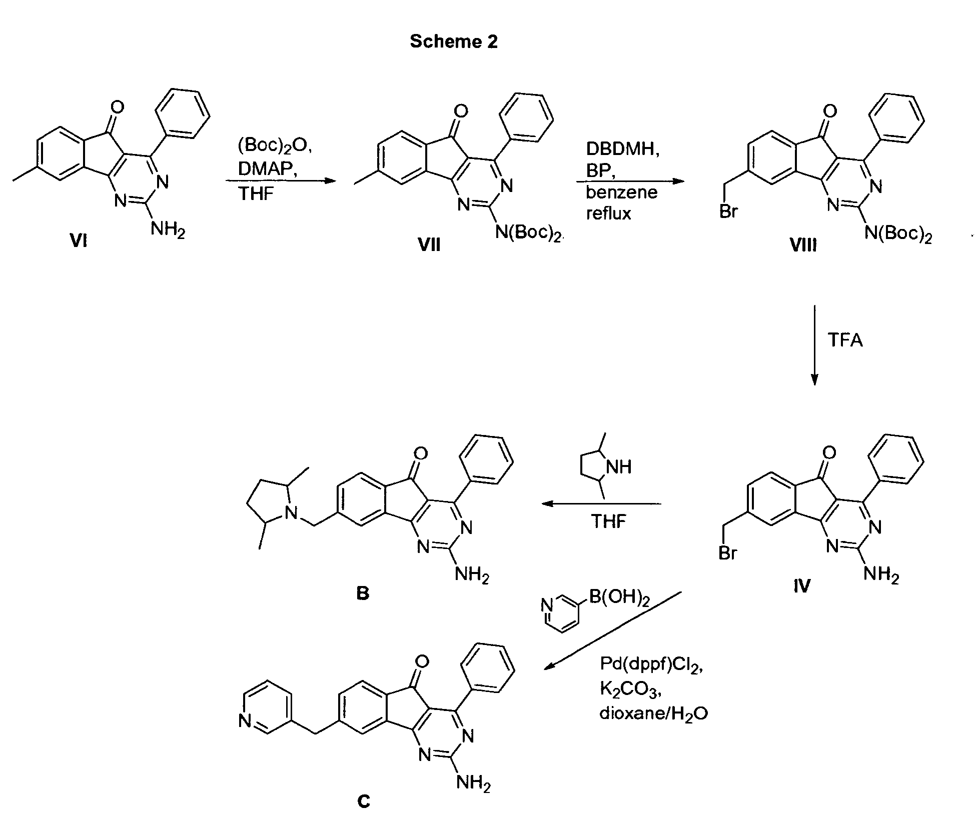 Scheme 2 illustrates the synthetic route leading to compounds **B** and **C.** Starting with amino pyrimidine **VI** and following the path indicated by the arrows, protection of the amino (NH₂) can be accomplished using di-*tert*-butyl dicarbonate ((Boc)₂O) in THF in the presence of dimethylamino pyridine (DMAP). The resulting di-Boc protected **VII** can undergo a radical initiated benzylic bromination using 1,3-dibromo-5,5-dimethylhydantoin (DBDMH) and benzoyl peroxide (BP) in refluxing benzene to give the corresponding benzyl bromide **VIII.** Benzyl bromide **VIII** can then be deprotected with TFA to give the corresponding amino pyrimidine **IV.** Finally, the benzyl bromide **IV** can be alkylated 2,5-dimethylpyrrolidine to give compound **B.** Alternatively benzyl bromide **IV** can also undergo a Suzuki coupling with 3-pyridylboronic acid to afford compound **C.**

### Example B: step a

**(VII):** Neat dimethylamino pyridine (850 mg, 7.0 mmol) was added to a THF solution (300 mL) of **VI** (20.0 g, 69.7 mmol) and (Boc)₂O (38.0 g, 174.2 mmol). After 2 h the mixture was diluted with EtOAc and then washed with water and brine, dried (Na₂SO₄) and concentrated. The resulting solid was suspended in EtOAc (250 mL) and filtered. The solid was washed with EtOAc (2 x 100 mL) then dried *in vacuo* to give 25.6 g of **VII.**

### Example B: step b

**(VIII): VII** (25.6 g, 52.6 mmol) was completely dissolved in benzene (200 mL) by warming then dibromodimethyl hydantoin (8.3 g, 28.9 mmol) and benzoyl peroxide (1.0 g, 4.2 mmol) were added sequentially. The mixture was heated to reflux for 16 h. The solution was then cooled to rt, diluted with EtOAc and washed with saturated aqueous NaHCO₃, water and brine. The solution was dried (Na₂SO₄), concentrated and purified via column chromatography (5-20 % EtOAc/ heptane). First chromatography afforded 6 g of **VIII** that contained ∼ 10% **VII** and a second chromatography gave an additional 12 g of **VIII** containing 10% **VII.**

### Example B: step c

**(IV):** The bromide **VIII** (1.3 g, 2.3 mmol) was then stirred in 8 mL CH₂Cl₂/trifluoroacetic acid (TFA) (1:1). After 3 h the mixture was concentrated, neutralized with saturated aqueous NaHCO₃ and filtered to give 750 mg of **IV** as a light yellow solid that was used without further purification.

### Example B: step d

### 2-Amino-8-(2,5-dimethyl-pyrrolidin-1-ylmethyl)-4-phenyl-indeno[1,2-d]pyrimidin-5-one

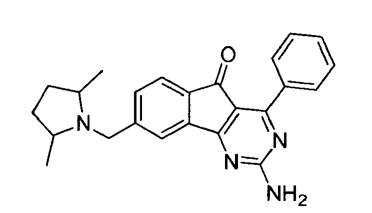

**(B):** Neat 2,5-dimethylpyrrolidine-mix of *cis-* and *trans*-isomers (1.1 mL, 9:3 mmol) was added to a THF solution (20 mL) of bromide **IV** (1.7 g, 4.6 mmol) and triethyl amine (Et₃N) (1.3 mL, 9.3 mmol) and the resulting mixture was heated to 75 °C. After 3 h the mixture was concentrated in vacuo, dissolved into CH₂Cl₂. The mixture was washed with saturated aqueous NaHCO₃ and brine (2x), dried (Na₂SO₄), concentrated and purified via column chromatography to give 1.0 g of **B. B** was dissolved in CH₂Cl₂ and added dropwise to an excess of HCl in ether. The resulting precipitate was filtered off to give 1.1 g of **B** as the di-HCl salt. 1H NMR (300 MHz, CHLOROFORM-*d*) δ ppm 1.04 (d, *J*=6.03 Hz, 6 H) 1.34 - 1.51 (m, 2 H) 1.74 - 1.93 (m, 2 H) 2.52 - 2.76 (m, 2 H) 3.79 (s, 2 H) 5.80 (br. s., 2 H) 7.42 - 7.58 (m, 4 H) 7.61 - 7.70 (m, 1 H) 7.84 (s, 1 H) 8.06 (dd, *J*=7.54, 2.26 Hz, 2 H); MS m/e 385 (M+H).

### Example C: step a

### 2-Amino-4-phenyl-8-pyridin-3-ylmethyl-indeno[1,2-d] pyrimidin-5-one

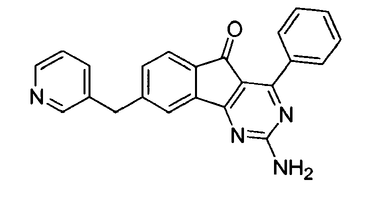

**(C):** A dioxane/water (4:1) solution (5 mL) of bromide **IV** (175 mg, 0.48 mmol), 3-pyridylboronic acid (94 mg, 0.77 mmol), K₂CO₃ (133 mg, 0.96 mmol) and Pd(dppf)Cl₂ (39 mg, 0.05 mmol) was heated in the microwave at 120 °C for 10 minutes. The resulting mixture was diluted with THF and EtOAc, washed with water and brine, dried (Na₂SO₄), concentrated and purified to give 112 mg of **C**. **C** was dissolved in THF added dropwise to an excess of HCl in ether. The resulting precipitate was filtered off to give 115 mg of **C** as the di-HCl salt. 1H NMR (400 MHz, DMSO-*d₆*) δ ppm 4.40 (s, 2 H) 7.42 - 7.60 (m, 3 H) 7.65 (s, 2 H) 7.73 (s, 1 H) 7.89 - 8.13 (m, 5 H) 8.55 (d, J=8.07 Hz, 1 H) 8.84 (d, *J=*5.14 Hz, 1 H) 9.02 (s, 1 H); MS m/e 365 (M+H).

### BIOLOGICAL ASSAYS AND ACTIVITY LIGAND BINDING ASSAY FOR ADENOSINE A2a RECEPTOR

Ligand binding assay of adenosine A2a receptor was performed using plasma membrane of HEK293 cells containing human A2a adenosine receptor (PerkinElmer, RB-HA2a) and radioligand 13H] CGS21680 (PerkinElmer, NET1021). Assay was set up in 96-well polypropylene plate in total volume of 200,uL by sequentially adding 20 pL1:20 diluted membrane, pLassay buffer (50 mM Tris HCI, pH7.4 10 mM MgCI2, 1 mM EDTA) containing [3H] CGS2168O, 50,uL diluted compound (4X) or vehicle control in assay buffer. Nonspecific binding was determined by 80 mM NECA. Reaction was carried out at room temperature for 2 hours before filtering through 96 well GF/C filter plate pre-soaked in 50 mM Tris HCI, pH7.4 containing 0.3% polyethylenimine. Plates were then washed 5 times with cold 50 mM Tris HCI, pH7.4, dried and sealed at the bottom. Microscintillation fluid 30,ul was added to each well and the top sealed. Plates were counted on Packard Topcount for [3H]. Data was analyzed in Microsoft Excel and GraphPad Prism programs. (Varani, K.; Gessi, S.; Dalpiaz, A.; Borea, P.A. British Journal of Pharmacology, 1996, 117, 1693) Adenosine A2a Receptor Functional Assay CHO-K1 cells overexpressing human adenosine A2a receptors and containing cAMP-inducible beta-galactosidase reporter gene were seeded at 40-50K/well into 96-well tissue culture plates and cultured for two days. On assay day, cells were washed once with 200 pL assay medium (F-12 nutrient mixture/0.1 % BSA). For agonist assay, adenosine A2a receptor agonist NECA was subsequently added and cell incubated at 37°C, 5% CO2 for 5 hrs before stopping reaction. In the case of antagonist assay, cells were incubated with antagonists for 5 minutes at R.T. followed by addition of 50 nM NECA. Cells were then incubated at 37°C, 5% CO2 for 5 hrs before stopping experiments by washing cells with PBS twice. 50,uL 1X lysis buffer (Promega, 5X stock solution, needs to be diluted to 1X before use) was added to each well and plates frozen at-20°C. For,B-galactosidase enzyme calorimetric assay, plates were thawed out at room temperature and 50,uL 2X assay buffer (Promega) added to each well. Color was allowed to develop at 37°C for 1 h or until reasonable signal appeared. Reaction was then stopped with 150 AL 1 M sodium carbonate. Plates were counted at 405 nm on Vmax Machine (Molecular Devices). Data was analyzed in Microsoft Excel and GraphPad Prism programs. (Chen, W.B.; Shields, T.S.; Cone, R. D. Analytical Biochemistry, 1995, 226, 349; Stiles, G. Journal of Biological Chemistry, 1992, 267, 6451) ; Haloperidol-induced catalepsy study in C57b1/6 mice Mature male C57b1/6 mice (9-12 week old from ACE) were housed two per cage in a rodent room. Room temperature was maintained at 64-79 degrees and humidity at 30-70% and room lighting at 12 hrs lighV12 hrs dark cycle. On the study day, mice were transferred to the study room. The mice were injected subcutaneously with haloperidol (Sigma H1512, 1.0 mg/ml made in 0.3% tartaric acid, then diluted to 0.2 mg/ml with saline) or vehicle at 1.5 mg/kg, 7.5ml/kg. The mice were then placed in their home cages with access to water and food. 30 minutes later, the mice were orally dosed with vehicle (0.3% Tween 80 in saline) or compounds at 10 mg/kg, 10 ml/kg (compounds, 1 mg/ml, made in 0.3% Tween 80 in saline, sonicated to obtain a uniform suspension). The mice were then placed in their home cages with access to water and food. 1 hour afler oral dose, the catalepsy test was performed. A vertical metal-wire grid (1.0 cm squares) was used for the test.

The mice were placed on the grid and given a few seconds to settle down and their immobility time was recorded until the mice moved their back paw(s). The mice were removed gently from the grid and put back on the grid and their immobility time was counted again. The measurement was repeated three times. The average of three measurements was used for data analysis.

### A2a ASSAY DATA

| Compound | A₂a cell-based functional Ki | A₁ cell-based functional Ki |
|---|---|---|
| A | 7.7 nM | 49.6 nM |
| B | 13.1 nM | 37.3 nM |
| C | 0.4 nM | 4.4 nM |

### AMES ASSAY CONDITIONS

The purpose of this study was to assess, in vitro, the ability of compounds of the present invention to induce reverse-point mutations in bacteria when treated in the presence and absence of a microsomal activation system.

Compounds were tested in a bacterial/microsomal activation plate incorporation assay using *Salmonella typhimurium* strains TA98, TA100, TA1535, TA1537, and *Escherichia coli* strain WP₂uvrA. This study included tests in the absence (buffer) and presence of metabolic activation by an Aroclor^{®} 1254-induced rat liver microsomal preparation (S9 mix). Compounds were tested in all strains under both metabolic conditions at doses of 5, 10, 25, 50, 100, 250, 500, 1000, 2500, and 5000 µg per plate. Mutations were detected by phenotypic reversion to amino acid prototrophy (histidine or tryptophan for S. *typhimurium* or *E*. *coli* respectively). A test article would be considered positive (mutagenic), if it induces a dose dependent increase in revertant frequency to at least 2-fold that observed in the appropriate concurrent vehicle control (3-fold for TA1535 and TA1537). In addition, the response should be reproducible. Toxicity was detected by a dose dependent decrease in colony counts and/or reduced/absent bacterial lawns. Vehicle treated plates served as the standards for comparison for both mutation and toxicity. Positive control plates were used to assure the functionality of the test system. Acceptable negative control and positive indicator results were obtained for all strains in the absence and the presence of S9 mix. This assured that the test system was functioning and responsive.

### AMES ASSAY RESULTS

The following results demostrate the desirable AMES-negative quality for the three compounds of the present invention. Not all of the compounds tested in the assay were found to be AMES-negative. For comparison, four similar molecules are shown which possess the undesirable AMES-positive quality. For this assay, AMES-negative is considered to be a desirable quality.

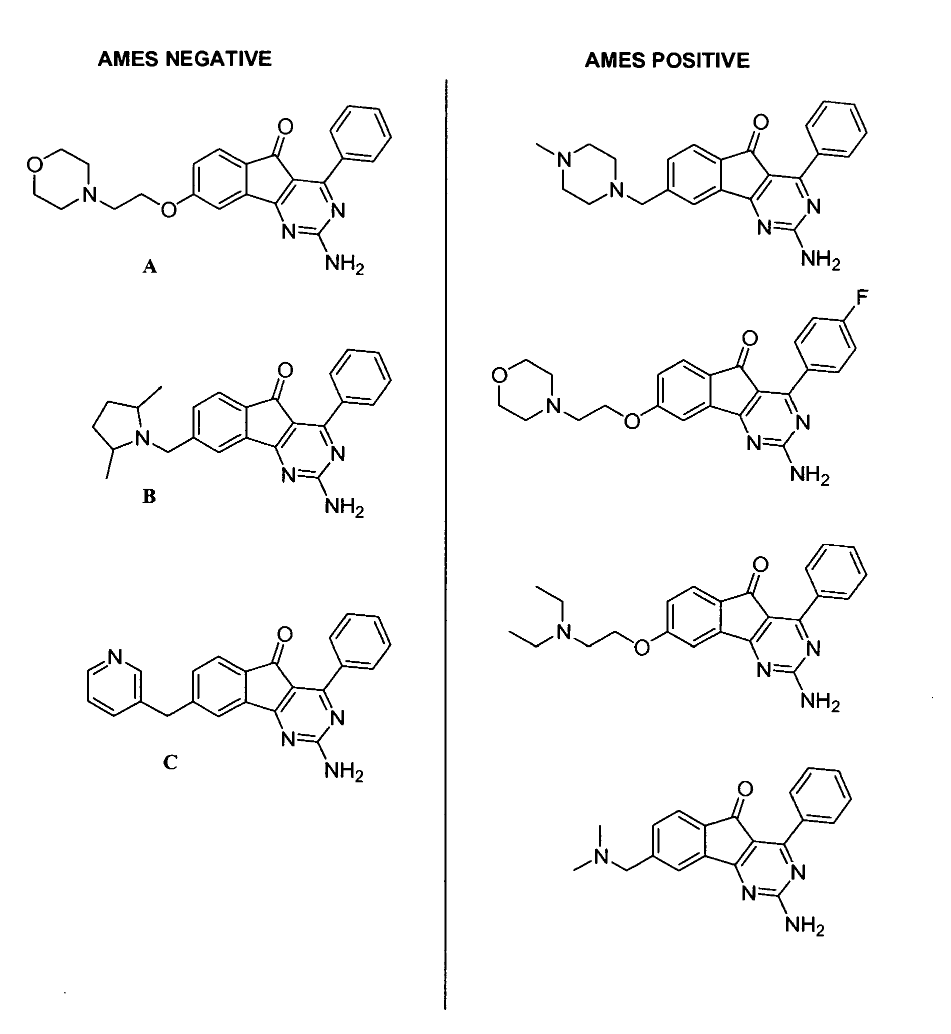

## Claims

1. A compound, which is: 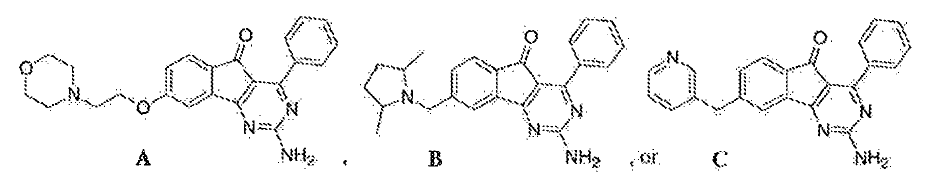 and solvates, hydrates, tautomers, and pharmaceutically acceptable salts thereof.

2. A compound of Claim 1, which is: 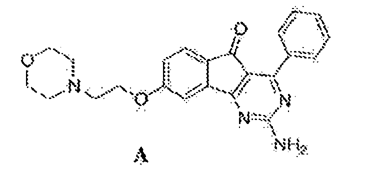 and solvates, hydrates, tautomers, and pharmaceutically acceptable salts thereof.

3. A compound of Claim 1, which is: 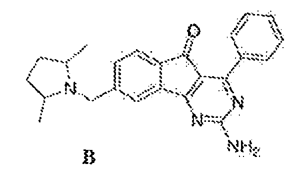 and solvates, hydrates, tautomers, and pharmaceutically acceptable salts thereof.

4. A compound of Claim 1, which is: 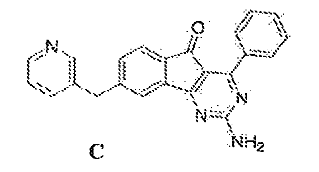 and solvates, hydrates, tautomers, and pharmaceutically acceptable salts thereof.

5. A pharmaceutical composition comprising a compound of claim 1; and a pharmaceutically acceptable carrier.

6. A compound according to claim 1 for use in a method of treating a subject having a disorder ameliorated by antagonizing Adenosine A2a receptors in appropriate cells in the subject, which comprises administering to the subject a therapeutically effective dose of a compound of claim 1.

7. A compound according to claim 1 for use in a method of preventing a disorder ameliorated by antagonizing Adenosine. A2a receptors in appropriate cells in the subject, comprising administering to the subject a prophylactically effective dose of a compound of claim 1 either preceding or subsequent to an event anticipated to cause a disorder ameliorated by antagonizing Adenosine A2a receptors in appropriate cells in the subject.

8. A compound according to claim 6 or claim 7, wherein said use comprises administering to the subject a therapeutically or prophylactically effective dose of the pharmaceutical composition of Claim 5.

9. A compound according to claim 6, wherein the disorder is a neurodegenerative disorder or a movement disorder.

10. A compound according to claim 6 or claim 7, wherein the disorder is selected from the group consisting of Parkinson's Disease, Huntington's Disease, Multiple System Atrophy, Corticobasal Degeneration, Alzheimer's Disease, and Senile Dementia.

11. A compound according to claim 7, wherein the disorder is a neurodegenerative disorder or a movement disorder.

12. A compound according to Claim 6, wherein the disorder is addiction.

13. A compound according to Claim 6, wherein the disorder is Attention deficit Hyperactivity Disorder (ADHD).

14. A compound according to Claim 6, wherein the disorder is depression.

15. A compound according to Claim 6, wherein the disorder is anxiety.

## Patentansprüche

1. Verbindungen, bei denen es sich um: 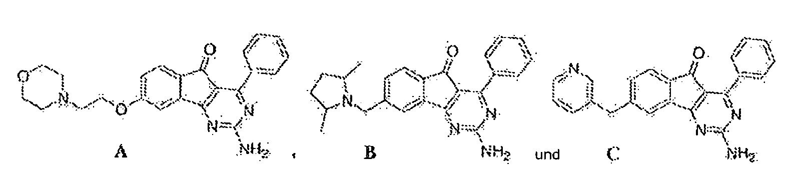 und deren Solvate, Hydrate, Tautomere und pharmazeutisch unbedenkliche Salze handelt.

2. Verbindungen nach Anspruch 1, bei denen es sich um: 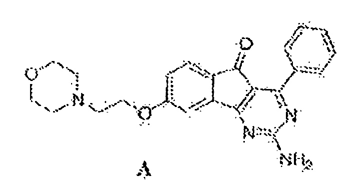 und deren Solvate, Hydrate, Tautomere und pharmazeutisch unbedenkliche Salze handelt.

3. Verbindungen nach Anspruch 1, bei denen es sich um: 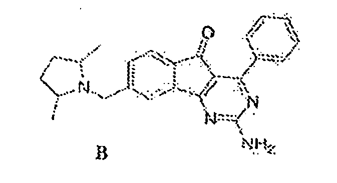 und deren Solvate, Hydrate, Tautomere und pharmazeutisch unbedenkliche Salze handelt.

4. Verbindungen nach Anspruch 1, bei denen es sich um: 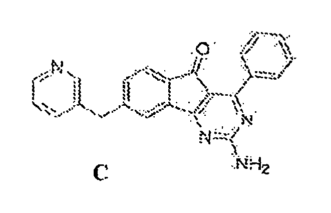 und deren Solvate, Hydrate, Tautomere und pharmazeutisch unbedenkliche Salze handelt.

5. Pharmazeutische Zusammensetzung, enthaltend eine Verbindung nach Anspruch 1 und einen pharmazeutisch unbedenklichen Träger.

6. Verbindungen nach Anspruch 1 zur Verwendung in einem Verfahren zur Behandlung eines Patienten, der an einer Erkrankung leidet, die durch Antagonisieren von Adenosin-A2a-Rezeptoren in entsprechenden Zellen im Patienten gelindert wird, wobei man dem Patienten eine therapeutisch wirksame Dosis einer Verbindung nach Anspruch 1 verabreicht.

7. Verbindungen nach Anspruch 1 zur Verwendung in einem Verfahren zur Prävention einer Erkrankung, die durch Antagonisieren von Adenosin-A2a-Rezeptoren in entsprechenden Zellen in dem Patienten gelindert wird, wobei man dem Patienten eine prophylaktisch wirksame Dosis einer Verbindung nach Anspruch 1 verabreicht, und zwar vor oder nach einem Ereignis, von dem angenommen wird, dass es zu einer Erkrankung führt, die durch Antagonisieren von Adenosin-A2a-Rezeptoren in entsprechenden Zellen im Patienten gelindert wird.

8. Verbindungen nach Anspruch 6 oder 7, wobei man bei der Verwendung dem Patienten eine therapeutisch oder prophylaktisch wirksame Dosis der pharmazeutischen Zusammensetzung nach Anspruch 5 verabreicht.

9. Verbindungen nach Anspruch 6, wobei es sich bei der Erkrankung um eine neurodegenerative Erkrankung oder eine Bewegungsstörung handelt.

10. Verbindungen nach Anspruch 6 oder 7, wobei die Erkrankung ausgewählt ist aus der aus Parkinson-Krankheit, Huntington-Krankheit, Multipler Systematrophie, kortikobasaler Degeneration, Alzheimer-Krankheit und seniler Demenz bestehenden Gruppe.

11. Verbindungen nach Anspruch 7, wobei es sich bei der Erkrankung um eine neurodegenerative Erkrankung oder eine Bewegungsstörung handelt.

12. Verbindungen nach Anspruch 6, wobei es sich bei der Erkrankung um Sucht handelt.

13. Verbindungen nach Anspruch 6, wobei es sich bei der Erkrankung um Aufmerksamkeitsdefizit-/Hyperaktivitätsstörung (Attention Deficit Hyperactivity Disoder - ADHD) handelt.

14. Verbindungen nach Anspruch 6, wobei es sich bei der Erkrankung um Depression handelt.

15. Verbindungen nach Anspruch 6, wobei es sich bei der Erkrankung um Angst handelt.

## Revendications

1. Composé, qui est : 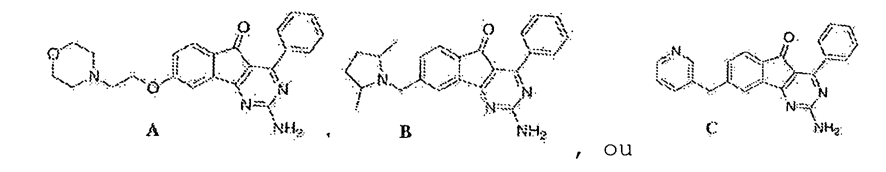 et les solvates, les hydrates, les tautomères et les sels pharmaceutiquement acceptables de celui-ci.

2. Composé selon la revendication 1, qui est : 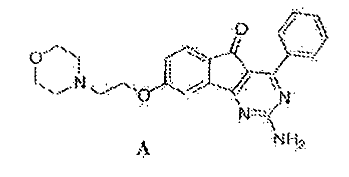 et les solvates, les hydrates, les tautomères et les sels pharmaceutiquement acceptables de celui-ci.

3. Composé selon la revendication 1, qui est : 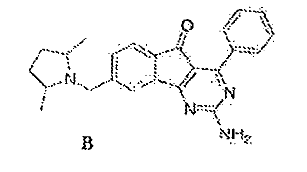 et les solvates, les hydrates, les tautomères et les sels pharmaceutiquement acceptables de celui-ci.

4. Composé selon la revendication 1, qui est : 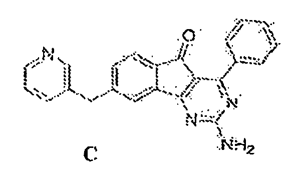 et les solvates, les hydrates, les tautomères et les sels pharmaceutiquement acceptables de celui-ci.

5. Composition pharmaceutique comprenant un composé selon la revendication 1, et un support pharmaceutiquement acceptable.

6. Composé selon la revendication 1, destiné à être utilisé dans une méthode de traitement d'un sujet ayant un trouble amélioré en antagonisant les récepteurs A2a d'adénosine dans les cellules appropriées chez le sujet, qui comprend l'administration au sujet d'une dose thérapeutiquement efficace d'un composé selon la revendication 1.

7. Composé selon la revendication 1, destiné à être utilisé dans une méthode de prévention d'un trouble amélioré en antagonisant les récepteurs A2a d'adénosine dans les cellules appropriées chez le sujet, comprenant l'administration au sujet d'une dose prophylactiquement efficace d'un composé selon la revendication 1 soit avant soit après un événement que l'on pense causer un trouble amélioré en antagonisant les récepteurs A2a d'adénosine dans les cellules appropriées chez le sujet.

8. Composé selon la revendication 6 ou la revendication 7, **caractérisé en ce que** ladite utilisation comprend l'administration au sujet d'une dose thérapeutiquement ou prophylactiquement efficace de la composition pharmaceutique selon la revendication 5.

9. Composé selon la revendication 6, **caractérisé en ce que** le trouble est un trouble neurodégénératif ou un trouble du mouvement.

10. Composé selon la revendication 6 ou la revendication 7, **caractérisé en ce que** le trouble est choisi dans le groupe constitué de la maladie de Parkinson, de la maladie de Huntington, de l'atrophie multisystémique, de la dégénérescence corticobasale, de la maladie d'Alzheimer et de la démence sénile.

11. Composé selon la revendication 7, **caractérisé en ce que** le trouble est un trouble neurodégénératif ou un trouble du mouvement.

12. Composé selon la revendication 6, **caractérisé en ce que** le trouble est la dépendance.

13. Composé selon la revendication 6, **caractérisé en ce que** le trouble est le trouble de l'attention et de l'hyperactivité (TDAH).

14. Composé selon la revendication 6, **caractérisé en ce que** le trouble est la dépression.

15. Composé selon la revendication 6, **caractérisé en ce que** le trouble est l'anxiété.
